(19) Europäisches Patentamt
European Patent Office
Office européen des brevets

(11) Veröffentlichungsnummer: **0 374 620**
**A2**

# (12) EUROPÄISCHE PATENTANMELDUNG

(21) Anmeldenummer: **89122651.6**

(51) Int. Cl.[5]: **G01N 33/52**

(22) Anmeldetag: **08.12.89**

(30) Priorität: **19.12.88 DE 3842657**

(43) Veröffentlichungstag der Anmeldung:
**27.06.90 Patentblatt 90/26**

(84) Benannte Vertragsstaaten:
**BE CH DE FR GB IT LI NL SE**

(71) Anmelder: **MERCK PATENT GESELLSCHAFT MIT BESCHRÄNKTER HAFTUNG**
**Frankfurter Strasse 250 Postfach 4119**
**D-6100 Darmstadt(DE)**

(72) Erfinder: **Dreher, Michael, Dr.**
**Dresdner Strasse 7**
**D-6108 Weiterstadt(DE)**
Erfinder: **Hirsch, Heinz**
**Am Kirchpfad 59**
**D-6108 Weiterstadt(DE)**

## (54) Verfahren und Mittel zur Bestimmung von Maleimidgruppen.

(57) Die Erfindung betrifft ein Verfahren und Mittel zur Bestimmung von Maleimidgrupen. Das Verfahren ist dadurch gekennzeichnet, daß man eine Maleimidgruppen enthaltende Probe mit einem Thiolgruppen enhaltenden Reagenz und anschließend mit einem mit Thiolgruppen reagierenden Chromogen oder Fluorogen umsetzt und die Intensitätsänderung mißt.

EP 0 374 620 A2

## Verfahren und Mittel zur Bestimmung von Maleimidgruppen

Die Erfindung betrifft ein Verfahren und Mittel zur Bestimmung von Maleimidgruppen, insbesondere die Bestimmung von an Proteine (Enzyme, Antigene, Antikörper) gebundener reaktiver Maleimidgruppen.

Seit Mitte der siebziger Jahre wurden zahlreiche sogenannte Heterocrosslinker entwickelt, mit dem Ziel, Proteine über unterschiedliche reaktive Gruppen, z.B. Aminogruppen, Carboxylgruppen, Hydroxylgruppen und Thiolgruppen, miteinander kovalent zu binden. Besonderes Interesse haben diese Heterocrosslinker auf dem Gebiet der Immunoassayreagenzien erlangt. Speziell Maleimidgruppen tragende Heterocrosslinker sind hierbei hervorzuheben, weil sie die Eigenschaft besitzen, spezifisch mit Thiolgruppen zu reagieren. Auf diese Weise können Antikörper-Fragmente, z.B. Fab'-Fragmente, die freie Thiolgruppen enthalten, selektiv an andere Proteine, z.B. Enzyme, gebunden werden.

Solche Maleimidgruppen tragende Crosslinker und Heterocrosslinker sind z.B. m-Maleimidobenzoyl-N-hydroxysuccinimidester (MBS), m-Maleimidobenzoyl-N-hydroxysulfosuccinimidester (Sulfo-MBS), Succinimidyl-4-(N-maleimidomethyl)-cyclohexan-1-carboxylat (SMCC), Sulfosuccinimidyl-4-(N-maleimidomethyl)-cyclohexan-1-carboxylat (Sulfo-SMCC), Succinimidyl-4-(p-maleimidophenyl)-butyrat (SMPB), Sulfosuccinimidyl-4-(p-maleimidophenyl)-butyrat (Sulfo-SMPB), Bis-maleimidohexan (BMH), N-(4-diazophenyl)-maleimid, N-(ß-diazophenyl)-ethylmaleimid.

Mit Hilfe dieser Heterocrosslinker können Thiolgruppen an Aminogruppen gebunden werden. N-(4-diazophenyl)-maleimid und N-(ß-diazophenyl)-ethylmaleimid erlauben darüber hinaus auch die Bindung von Thiolgruppen an Imidazol-, Phenol-, Indol- und andere Gruppen.

Prinzipiell erfolgen diese Reaktionen nach dem folgenden Schema:

$$\text{R-NH}_2 + \text{Succ-O-CO-X-Mal} \rightarrow \underset{\text{(I)}}{\text{R-NH-CO-X-Mal}} + \text{Succ-OH} \quad \text{(1. Schritt)}$$

$$\text{R-NH-CO-X-Mal} + \text{HS-R'} \rightarrow \underset{\text{(II)}}{\text{R-NH-CO-X-MalH-S-R'}} \quad \text{(2. Schritt)}$$

wobei $R-NH_2$ = primäre Aminogruppen tragendes Protein; Mal = Maleimidgruppe; Succ = Succinimidylgruppe; $R'$-SH = Thiolgruppen tragendes Protein; X = Spacer bedeuten.

Überraschenderweise wurde gefunden, daß es für die Herstellung wirksamer Immunoassayreagenzien entscheidend ist, Anzahl und Funktionalität der an den Spacer gebundenen Maleimidgruppen im Zwischenprodukt I der obigen Gleichung zu kennen, bevor die Umsetzung mit z.B. Antikörperfragmenten erfolgt. Dadurch lassen sich kostenintensive Fehlproduktionen vermeiden.

Der vorliegenden Erfindung liegt die Aufgabe zugrunde, ein Verfahren und Mittel zur Verfügung zu stellen, mit dem der Nachweis der Anzahl funktionsfähiger, reaktiver Maleimidgruppen im obigen Zwischenprodukt I durchgeführt werden kann.

Gegenstand der Erfindung ist ein Verfahren zur Bestimmung von Maleimidgruppen, das dadurch gekennzeichnet ist, daß man eine Maleimidgruppen enthaltende Probe mit einem Thiolgruppen enthaltenden Reagenz und anschließend mit einem mit Thiolgruppen reagierenden Chromogen oder Fluorogen umsetzt und die Intensitätsänderung mißt.

Ein weiterer Gegenstand der Erfindung ist ein Mittel zur Bestimmung von Maleimidgruppen, enthaltend ein Thiolgruppen enthaltendes Reagenz und ein mit Thiolgruppen reagierendes Chromogen oder Fluorogen.

Die direkte Bestimmung von an Proteine gebundenen Maleimidgruppen mit Chromogenen oder Fluorogenen erfordert eine chromatographische Trennng. Das erfindungsgemäße Verfahren erfolgt dagegen in einer homogenen, d.h. einphasigen Arbeitsweise.

Die Bestimmung von Maleimidgruppen erfolgt in der Weise, daß man z.B. eine Lösung eines Maleimidgruppen tragenden Proteins mit einer Lösung eines Mercaptoalkanols versetzt und inkubiert. Anschließend wird eine Lösung eines Chromogens oder Fluorogens zugesetzt, das mit Thiolgruppen reagiert. Überschüssiges, d.h. nicht von den Maleimidgruppen gebundenes Mercaptoalkanol bewirkt die Bildung eines Farbstoffs bzw. eine Flureszenzänderung, dessen Intensität gemessen wird. Das Verfahren kann sowohl als Endpunktmethode als auch als kinetische Methode durchgeführt werden, wobei die Geschwindigkeit der Bildung des Farbstoffs bzw. der Fluoreszenzänderung gemessen wird.

Geeignete Thiolgruppen enthaltende Reagenzien sind Mercaptoalkanole wie Mercaptoethanol, Mercaptopropanol, Mercaptoalkylamine wie Cysteamin, Mercaptopropylamin, Mercaptoaminosäuren wie Cystein, Mercaptocarbonsäuren wie Mercaptoessigsäure, Mercaptobernsteinsäure, 2-Thiobenzoesäure, vorzugsweise Mercaptoethanol. Das Thiolgruppen enthaltende Reagenz muß in Bezug auf das Maleimidgruppen tragende Protein in 2- bis 10-fachem molaren Überschuß vorliegen, vorzugsweise in 4- bis 6-fachem Überschuß.

Als mit Thiolgruppen reagierende Chromogene eignen sich z.B. 2,2′-Dinitro-5,5′-dithio-dibenzoesäure, Bis(p-nitrophenyl)-disulfid, 2,2′-Dipyridyl-disulfid, vorzugsweise 2,2′-Dinitro-5,5′-dithio-dibenzoesäure,die unter der Bezeichnung Ellmans-Reagenz seit langem bekannt ist (Arch. Biochem. Biophys. 82, 70 (1959)). In schwach basischem Milieu (pH 8-9) bildet sich mit Ellmans-Reagenz durch reduktive Spaltung der Disulfidbrücke das intensiv gelbe 2-Nitro-5-thiobenzoat, dessen Farbintensität im Bereich von 400-450 nm ein Maß für die Anzahl an Thiolgruppen ist. Die Konzentration des Chromogens sollte im Bereich von 0,1 bis 1 mM liegen, vorzugsweise bei 0,2 mM.

Als fluorogene Substanzen eignen sich z.B. (o-Nitroanilin-N-ethyldithio)-2-pyridyl-5-thioureido-N′-(5-fluorescein), 4-Jodacetylamido-1-naphthol, vorzugsweise (o-Nitroanilin-N-ethyldithio)-2-pyridyl-5-thioureido-N′-(5-fluorescein). Prinzipiell verläuft die Methode analog dem von Ellman beschriebenen Verfahren. Durch Umsetzung des Fluoresceinderivats mit Thiolgruppen enthaltenden Verbindungen wird die Fluoreszenz des Fluores ceinderivats stark erhöht. Die Fluoreszenz-Ausbeute ist, bezogen auf einen Standard, ein Maß für die Anzahl von Thiolgruppen. Die Konzentration des Fluorogens sollte im Bereich von 0,1 bis 1 mM liegen, vorzugsweise bei 0,2 mM.

Das Verfahren und Mittel eignet sich allgemein für die Bestimmung von Maleimid bzw. Maleimidgruppen enthaltenden Verbindungen. Es eignet sich vorzugsweise für die Bestimmung der Sensitivität von Maleimidgruppen enthaltenden Immunoassayreagenzien, d.h. für die Qualitätskontrolle derartiger Reagenzien.

Beispiel 1

Bestimmung von an Protein gebundenen Maleimidgruppen

Eine Lösung von Maleimidgruppen tragender Meerrettich-Peroxidase (0,062 mM) wird mit einer Lösung von Mercaptoethanol versetzt. Nach einer Inkubationszeit von 10 min wird eine Lösng von Ellmans Reagenz (2,2′-Dinitro-5,5′-dithio-dibenzoesäure ) zugesetzt. Überschüssiges, d.h. nicht von Maleimidgruppen gebundenes Mercaptoethanol bewirkt die Bildung des orangegelben Farbstoffs 2-Nitro-5-thio-benzoat.

Die Farbintensität wird in einem Photometer bei 436 nm und Raumtemperatur gemessen. Die Farbe der Lösung nach der Reaktion ist mindestens eine Stunde stabil, so daß die Messung als Endpunktmessung erfolgen kann. Der gewählte Farbstoff hat den Vorteil, bei einer Wellenlänge zu absorbieren, wo Störungen durch Proteine kaum zu erwarten sind. Die Messung des Analysenleerwertes erfolgt nach dem nachstehenden Pipettierschema:

| | Standard | Standardleerwert | Analyse | Analysenleerwert |
|---|---|---|---|---|
| Lösung 1 | 100 | 150 | - | 50 |
| Lösung 2 | - | - | 100 | 100 |
| Lösung 3 | 50 | - | 50 | - |
| 10 Minuten Inkubation bei 37 °C | | | | |
| Lösung 4 | 2500 | 2500 | 2500 | 2500 |
| Nach 10 Minuten Inkubation Extinktion bei 436 nm gegen Wasser messen | | | | |
| | St | StL | A | AL |

Die Berechnung erfolgt nach folgender Gleichung:
[(St-StL)-(A-AL)] x 5 mM/(St-StL) x 2

Zusammensetzung der Lösungen:

Lösung 1 = Wasser
Lösung 2 = Analyse (Maleimidgruppen tragende Substanz)
Lösung 3 = Standard (Mercaptoethanol, 5 mM in entgastem Wasser)
Lösung 4 = Farbreagenz (Ellmans Reagenz, 0,2 mM in 0,05 M Natriumborat + 0,1 % Ascorbinsäure, pH = 8,0)

Beispiel 2

Bestimmung der Anzahl (bzw. Konzentration) von reaktiven Maleimidgruppen in verschiedenen Crosslinkern und Wiederfindung bei verschiedenen Verdünnungen

Da viele Crosslinker schlecht wasserlöslich sind, bzw. die Succinimidylgruppe durch Wasser rasch hydrolysiert wird, werden alle Substnzen in Dioxan gelöst.

PAT LOG 12/5 081288

Probe 1: 8,60 mg Succinimidyl-4-(p-maleimidophenyl)butyrat werden in 9,65 ml Dioxan gelöst (2,5 mM).

Probe 2: 3,34 mg m-Maleimidobenzoyl-N-hydroxosuccinimidester werden in 4,25 ml Dioxan gelöst (2,5 mM).

Probe 3: 3,80 mg Bismaleimidohexan werden in 5,5 ml Dioxan gelöst (2,5 mM).

Der Test wird entsprechend dem Pipettierschema nach Beispiel 1 durchgeführt.

| Ergebnis (in mM, % Wiederfindung): | | | | | | | | |
|---|---|---|---|---|---|---|---|---|
| Probe 1 | | | Probe 2 | | | Probe 3 | | |
| ber. | gem. | % | ber. | gem. | % | ber. | gem. | % |
| 0.5 | 0,565 | 113 | 0,5 | 0,605 | 121 | 0,5 | 0,540 | 108 |
| 1,0 | 1,025 | 103 | 1,0 | 1,155 | 116 | 1,0 | 1,095 | 110 |
| 1,5 | 1,515 | 101 | 1,5 | 1,625 | 108 | SH-Gruppen verbraucht | | |
| 2,0 | 2,005 | 100 | 2,0 | 2,135 | 107 | | | |
| 2,5 | 2,475 | 99 | 2,5 | 2,490 | 100 | | | |

Das Ergebnis dieser Tabelle zeigt deutlich, daß selbst Maleimidgruppen unterschiedlicher Crosslinker richtig erfaßt werden. Der Test ist in einem ausreichend großen Konzentrationsintervall linear.

Beispiel 3

Bestimmung der Anzahl (bzw. Konzentration) von Maleimidgruppen an Meerrettich-Peroxidase

5,37 mg Maleimidgruppen tragende Meerrettich-Peroxidase (HRP) werden in 1 ml entgastem Wasser gelöst. Unter der Annahme eines Molekulargewichtes von 45000 wird somit eine Konzentration von 0,119 mM erreicht. Diese Stammlösung wird mit entgastem Wasser in mehreren Stufen verdünnt: 1 + 4, 2 + 3, 3 + 2, 4 + 1. Die Messung wird analog dem in Beispiel 1 angeführtem Pipettierschema durchgeführt.

| Ergebnis: | | |
|---|---|---|
| HRP-maleimid berechnet, mM | Maleimidgruppen gemessen, mM | Anzahl Maleimidgruppen pro Enzym |
| 0,0238 | 0,1965 | 8,26 |
| 0,0476 | 0,4760 | 10,00 |
| 0,0714 | 0,7600 | 10,64 |
| 0,0952 | 0,8000 | 8,40 |
| 0,1190 | 1,0150 | 8,53 |

Dieses Ergebnis zeigt deutlich, daß die Anwesenheit von Protein die Bestimmung nicht stört. Die Messung ist über einen ausreichend großen Konzentrationsbereich linear. Die Richtigkeit der Ergebnisse wird überprüft durch Bestimmung der Anzahl primärer Aminogruppen vor und nach der Umsetzung mit Heterocrosslinker. Im Mittel werden 10 Aminogruppen für die Bindung des Heterocrosslinkers verbraucht. Damit decken sich die Zahlenwerte beider Methoden sehr gut. Während das letzte Verfahren allerdings umständlich über eine zweimalige Bestimmung der primären Aminogruppen die Berechnung gebundener Maleimidgruppen erlaubt, erbringt die neue Methode gleiche Ergebnisse in kürzerer Zeit. Außerdem ist die neue Methode vor allem deshalb überlegen, weil hierbei ausschließlich aktive, d.h. zur Bindung von Thiolgruppen fähige Maleimidgruppen erfaßt werden.

## Ansprüche

1. Verfahren zur Bestimmung von Maleimidgrupen, dadurch gekennzeichnet, daß man eine Maleimidgruppen enthaltende Probe mit einem Thiolgruppen enhaltenden Reagenz und anschließend mit einem mit Thiolgruppen reagierenden Chromogen oder Fluorogen umsetzt und die Intensitätsänderung mißt.

2. Verfahren nach Anspruch 1, dadurch gekennzeichnet, daß man als Thiolgruppen enthaltendes Reagenz ein Mercaptoalkanol verwendet.

3. Verfahren nach den Ansprüchen 1 und 2, dadurch gekennzeichnet, daß man als Chromogen 2,2′-Dinitro-5,5′-dithio-dibenzoesäure verwendet.

4. Mittel zur Bestimmung von Maleimidgruppen, enthaltend ein Thiolgruppen enthaltendes Reagenz und ein mit Thiolgruppen reagierendes Chromogen oder Fluorogen.

5. Verwendung des Mittels nach Anspruch 4 zur Bestimmung der Sensitivität von Maleimidgruppen enthaltenden Immunoassayreagenzien.